(19) Europäisches Patentamt
European Patent Office
Office européen des brevets

(11) Numéro de publication: 0 168 305 B1

# (12) FASCICULE DE BREVET EUROPEEN

(45) Date de publication du fascicule du brevet: 22.04.87

(51) Int. Cl.⁴: C 07 C 69/734, C 07 C 67/343

(21) Numéro de dépôt: 85401217.6

(22) Date de dépôt: 19.06.85

## (54) Procédé de stabilisation des alkoxyalkylidènemalonates d'alkyles.

(30) Priorité: 25.06.84 FR 8410182

(43) Date de publication de la demande: 15.01.86 Bulletin 86/3

(45) Mention de la délivrance du brevet: 22.04.87 Bulletin 87/17

(84) Etats contractants désignés: AT BE CH DE FR GB IT LI LU NL SE

(56) Documents cités: FR - A - 2 273 793

(73) Titulaire: RHONE-POULENC SPECIALITES CHIMIQUES, "Les Miroirs" 18, Avenue d'Alsace, F-92400 Courbevoie (FR)

(72) Inventeur: Ratton, Serge, Hameau de Belmont Vaulx-Milieu, F-38090-Villefontaine (FR)

(74) Mandataire: Cazes, Jean-Marie et al, RHONE-POULENC INTERSERVICES Service Brevets Chimie 25, quai Paul Doumer, F-92408 Courbevoie Cédex (FR)

EP 0 168 305 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet un procédé d'obtention d'alkoxyalkylidènemalonates d'alkyle par condensation d'un orthoester avec un malonate d'alkyle.

Les composés organiques qui comportent un groupe alkoxyalkylidène répondant à la formule générale:

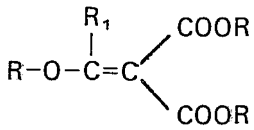

(I)

dans laquelle R représente un radical alkyle, $R_1$ un atome d'hydrogène, un radical alkyle ou un groupe phényle, sont des intermédiaires particulièrement recherchés en synthèse organique. Ainsi les alkoxyméthylènemalonates d'alkyle sont utilisés pour l'obtention d'anilinométhylènemalonates d'alkyle qui sont des intermédiaires de synthèse des quinoléines substituées telles que la dichloro-4,7 quinoléine ou la chloro-4 trifluorométhyl-7 quinoléine (cf. brevet français 950.883).

Le alkoxyalkylidènemalonates d'alkyle sont obtenus par condensation d'un malonate d'alkyle (malonate d'éthyle par exemple) avec un orthoester: orthoformiates, orthoacétates, orthobenzoates d'alkyle et en particulier de méthyle ou d'éthyle, cf. L. Claisen, Ber. *26* page 2729 et suivantes (1893) et Ann. *297* page 16 et suivantes (1897); P. Sah, J. Am. Chem. Soc. *53* page 1836 et suivantes (1931). Cette condensation qui peut être représentée par le schéma réactionnel suivant:

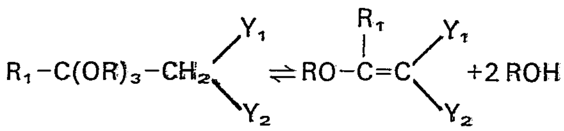

est en général conduite en présence de chlorure de zinc comme catalyseur et d'anhydride acétique comme agent de condensation. Cette réaction a fait l'objet de diverses études mécanistiques [cf. H. W. Post et al., J. Org. Chem. *2* page 260 et suivantes (1937) et R. C. Fuson et al., J. Org. Chem. *11* page 194 et suivantes (1946)] notamment dans le but d'améliorer les rendements en alkoxyméthylènemalonates (cf. R. C. Fuson et al., loc. cit.). Par la suite on a proposé divers perfectionnements au procédé de Claisen dans le but d'élever les rendements en alkoxyméthylènemalonates d'alkyle (en particulier vis-à-vis des orthoformiates) et la productivité de la réaction. Dans le brevet américain N° 2824121, on a proposé de réaliser la condensation en absence de chlorure de zinc, de remplacer l'anhydride acétique par une quantité d'acide acétique insuffisante pour bloquer sous forme d'acétate l'alcool formé au cours de la réaction et d'éliminer ce dernier par distillation au fur et à mesure de sa formation. En dépit de l'amélioration des résultats qu'il a procurés, ce procédé n'a pas conduit à des rendements suffisants vis-à-vis de l'orthoformiate. Dans la demande de brevet japonais, publiée sous le N° 4776/66, on a préconisé d'opérer en présence de catalyseurs métalliques (sels de zinc et de fer) en absence d'acide ou d'anhydride, au sein d'un solvant hydrocarboné (benzène, xylène, toluène) en assurant l'élimination de l'alcool formé par distillation azéotropique. Les rendements attribués à ce procédé n'ont pu être confirmés. Enfin dans la demande de brevet français N° 75/17.177, publiée sous le N° 2.273.793, on a proposé de prépare les alkoxyméthylènemalonates d'alkyle en faisant réagir un malonate d'alkyle avec un excès d'orthoformiate d'alkyle à 100-160° C, en présence d'un sel de zinc, d'aluminium ou de fer et en éliminant l'alcool résultant de la condensation au fur et à mesure de sa formation. Malgré l'amélioration des rendements qu'il apporte, ce procédé ne permet pas de dépasser un rendement théorique en alkoxyméthylènemalonate de 90% par rapport à l'orthoformiate.

On a encore constaté que les catalyseurs utilisés pour favoriser le déroulement de la réaction et notamment le zinc, le cadmium, la magnésium, le bismuth et le mercure dont l'emploi a été préconisé dans la demande de brevet français 84/02027 du 7 février 1984, favorisent la dégradation des alkoxyfalkylidènemalonates d'alkyles lors de leur distillation pour les séparer du milieu réactionnel et les purifier. Le taux de dégradation est évidemment fonction des conditions de distillation et dépend plus particulièrement de la température à laquelle est porté le mélange brut de condensation et de la durée de la distillation; ce taux de dégradation est également d'autant plus important que la quantité de catalyseur est élevée. D'une manière générale il peut atteindre de 2 à 5% en poids de l'alkoxyalkylidènemalonate produit au cours de la condensation. Lors de la distillation à l'échelle industrielle qui exige un taux de reflux important pour assurer une excellente pureté du produit, donc des durées de distillation élevées, le taux de dégradation du produit désiré atteint des valeurs maximales de l'ordre de 10%. Pour éviter cette influence néfaste du catalyseur sur la stabilité à chaud des alkoxyalkylidènemalonates il convient de l'éliminer de la masse réactionnelle préalablement à la distillation [cf. demande de brevet français 75/17177, publiée sous le N° 2273793]. Ce brevet enseigne d'ailleurs que les rendements en éthoxyméthylènemalonate d'éthyle décroissent lorsque la quantité, donc la concentration en acide de Lewis dans le milieu, augmente, ce qui est précisément le cas lors de la distillation. Cette élimination implique soit la filtration de la totalité de la masse réactionnelle sans aboutir à l'élimination de la totalité du catalyseur (une partie reste en solution dans le milieu), soit un lavage à l'eau acidifiée de la masse réactionnelle, opération complexe qui peut provoquer une perte de réactifs de départ et/ou d'alkoxyalkylidènemalonate par hydrolyse.

La présente invention a pour objet de résoudre le problème posé par la distillation des alkoxyalkylidènemalonates d'alkyle en présence des catalyseurs du type acide de Lewis utilisés au cours de la condensation de Claisen entre les orthoesters et les malonates d'alkyle; elle a plus généralement pour objet un moyen utilisable pour inhiber ou limiter l'action néfaste des acides de Lewis et notamment des sels (halogénures ou carboxylates) de zinc, de magnésium, de cadmium, de bismuth et de mercure sur la stabilité thermique des alkoxyalkylidènemalonates d'alkyles.

Plus spécifiquement, la présente invention a pour objet un procédé de stabilisation thermique des alkoxyalkylidènemalonates d'alkyles contenant des dérivés métalliques utilisés comme catalyseurs au cours de leur préparation par condensation entre un malonate et un orthoester, caractérisé en ce qu'on leur ajoute une quantité efficace d'un composé pris dans le groupe formé par les hydroxy-8 quinoléines et les phosphates acides organiques.

Le procédé selon l'invention permet de soumettre directement à la distillation, après addition d'une quantité efficace d'hydroxy-8 quinoléine ou de phosphate acide organique (dénommé ci-après stabilisant), la masse réactionnelle résultant de la condensation, en présence de dérivés métalliques et notamment d'acides de Lewis, des orthoesters avec les malonates d'alkyle pour séparer l'excès d'orthoester et l'alkoxyalkylidènemalonate d'alkyle. La présente invention a donc pour second objet un procédé de stabilisation thermique d'alkoxyalkylidènemalonates d'alkyles et en particulier de mélanges contenant notamment des orthoesters des alkoxyalkylidènemalonates d'alkyles d'alkyle et des acides de Lewis lors de leur distillation, caractérisé en ce que la distillation est conduite en présence d'une quantité efficace d'un composé pris dans le groupe formé par les hydroxy-8 quinoléines et les phosphates acides organiques.

Comme phosphates acides organiques on utilise des composés de formule générale:

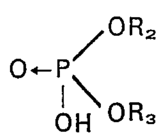 (I)

dans laquelle $R_2$ représente un radical hydrocarboné ayant de 1 à 20 atomes de carbone et $R_3$ un atome d'hydrogène ou un radical tel que $R_2$ et qui peut dans ce cas en être identique ou différent.

Plus spécifiquement, $R_2$ et $R_3$ peuvent représenter des radicaux alkyles ayant de 1 à 20 atomes de carbone tels que méthyle, éthyle, n-propyle, isopropyle, n-butyle, t-butyle, amyle, n-pentyle, éthyl-2 hexyle, n-hexyle, n-octyle, dodécyle, octadécyle; des radicaux cycloalkyles éventuellement substitués par des radicaux alkyles inférieurs (de 1 à 4 atomes de carbone) ayant de 5 à 12 atomes de carbone cycliques tels que les radicaux cyclopentyle, cyclohexyle, cycloheptyle, cyclodécyle; des radicaux arylalkyles comportant de 1 à 12 atomes de carbone dans la partie alkyle tels que les radicaux benzyle ou β-phényléthyle.

A titre d'exemples spécifiques de phosphates qui peuvent être utilisés dans le présent procédé on peut citer le phosphate de méthyle, le phosphate de diméthyle, le phosphate d'éthyle, le phosphate de diéthyle, le phosphate de di-n-propyle, de di-n-butyle, de di-isobutyle, de cyclohexyle, de dicyclohexyle, de benzyle, de β-phényléthyle. Les phosphates peuvent être utilisés sous forme de mélanges de mono, di ou triphosphates organiques et éventuellement de quantités résiduelles d'acide phosphorique, tels que ceux résultant de l'estérification des alcools par l'acide phosphorique. La présence d'un triphosphate, inerte quant au but poursuivi, n'est pas gênante.

On peut faire appel à toute hydroxy-8 quinoléine pour mettre en œuvre le procédé de l'invention. Plus spécifiquement on peut faire appel à des composés de formule:

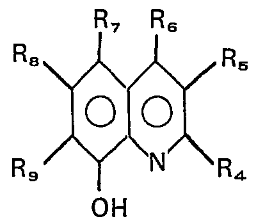 (II)

dans laquelle $R_4$ à $R_9$ identiques ou différents représentent un atome d'hydrogène ou des radicaux hydrocarbonés comportant de 1 à 20 et de préférence de 1 à 12 atomes de carbone, un atome d'halogène, un groupe $-SO_3H$, $-NO_2$, hydroxycarbonyle. Plus spécifiquement $R_4$ à $R_9$ représentent un radical alkyle comportant de 1 à 20 atomes de carbone tels que les radicaux méthyle, éthyle, n-propyle, isopropyle, n-butyle, isobutyle, n-pentyle, n-hexyle, n-heptyle, éthyl-2 hexyle, octyles, décyles; un radical cycloalkyle éventuellement substitué par 1 à 3 radicaux alkyle inférieurs tels que cyclopentyle, cyclohexyle, méthylcyclohexyle, éthylcyclohexyle; un radical aryle éventuellement substitué par 1 à 3 radicaux alkyles inférieurs tels que les radicaux phényle, toluyle, xylyle; un radical arylalkyle comportant de 1 à 4 atomes de carbone dans la partie alkyle tel que les radicaux benzyle, β-phényléthyle; un atome d'halogène tel que fluor, chlore ou brome; un radical alcényle ayant de 2 à 20 atomes de carbone, linéaires ou ramifiés, comportant une ou plusieurs doubles liaisons éthyléniques tel que vinyle, propène-2 yle, butène-2 yle, isobutényle, tétraméthyl-3,3,5,5 vinyl-1 hexyle. De préférence la somme des atomes de carbone des différents substituants $R_4$ à $R_9$ ne dépasse pas 20 et trois au plus des radicaux $R_4$ à $R_9$ représentent un groupe $-SO_3H$, $-NO_2$, $-COOH$ ou un atome d'halogène.

Comme exemples d'hydroxy-8 quinoléine on peut citer l'hydroxy-8 quinoléine, la méthyl-2 hydroxy-8 quinoléine, l'éthyl-3 hydroxy-8 quinoléine, l'éthyl-6 hydroxy-8 quinoléine, l'isopropyl-2 hydroxy-8 quinoléine, la n-pentyl-7 hydroxy-8 quinoléine, la cyclohexyl-2 hydroxy-8 quinoléine, la phényl-2 hydroxy-8 quinoléine, la benzyl-3 hydroxy-8 quinoléine, la méthyl-2 dichloro-5,7 hydroxy-8 quinoléine, la chloro-5 hydroxy-8 quinoléine, la méthyl-2 chloro-5 hydroxy-8 quinoléine, la trichloro-5,6,7 hydroxy-8 quinoléine-8, la dibromo-5,7 hydroxy-8 quinoléine, la méthyl-2 dibromo-5,7 hydroxy-8 quinoléine, la sulfonyl-5 hydroxy-8 quinoléine, la sulfonyl-7 hydroxy-8 quinoléine, la nitro-5 hydroxy-8 quinoléine, la nitro-5 méthyl-2 hydroxy-8 quinoléine, la nitro-5 chloro-2 hydroxy-8 quinoléine, l'hydroxycarbonyl-5 hydroxy-8 quinoléine, la (tétraméthyl-3,3,5,5 vinyl-1 hexyl)-2- hydroxy-8 quinoléine.

La quantité de stabilisant dépend de la nature et de la quantité de l'acide de Lewis présente dans l'alkoxyalkylidènemalonate d'alkyle et/ou dans le mélange résultant de la condensation de Claisen ainsi que de la nature du stabilisant. Elle peut être déterminée au moyen d'essais simples dans chaque cas particulier. En général cette quantité exprimée en moles de stabilisant par mole d'acide de Lewis est d'au moins 0,8 et de préférence au moins 1 mole par mole. Il n'y a pas de limite supérieure critique de la quantité de stabilisant, cependant il est sans intérêt pratique de mettre en œuvre plus de 10 moles par mole d'acide de Lewis.

Le procédé selon l'invention convient tout particulièrement bien à la distillation des mélanges provenant de la condensation d'un malonate d'alkyle avec un excès d'un orthoformiate d'alkyle en présence des acides de Lewis utilisés usuellement dans la condensation de Claisen et notamment des sels de zinc, tels que le chlorure de zinc, l'acétate de zinc, le propionate de zinc ou les sels de Cd, Mg, Bi et Hg tels que les halogénures, sulfates, nitrates, carbonates, phosphates, les carboxylates et les sulfonates. Pour des raisons pratiques on a recours de préférence aux halogénures (tout particulièrement aux chlorures et bromures) et aux carboxylates. Dans ce dernier cas on peut utiliser les sels de Cd, Hg, Bi et Mg de tout acide carboxylique tels que les acides mono- ou polycarboxyliques aliphatiques, cycloaliphatiques saturés ou non ou aromatiques. On peut citer plus particulièrement les sels des acides formique, acétique, propioniques, butyriques, pentanoïques, hexanoïques, octanoïques, dodécanoïques, hexadécanoïques, stéarique, oléique, ou des mélanges d'acides gras tels que l'acide naphténique, l'acide vendu sous la marque commerciale «acide versatique», l'acide benzoïque. En pratique on a recours de préférence aux chlorures et bromures de Cd, Hg, Bi et Mg et aux carboxylates d'acides aliphatiques inférieurs. A titre non limitatif, on peut citer le chlorure de cadmium, le bromure de cadmium, le chlorure et le bromure de magnésium, le chlorure et le bromure mercurique, le chlorure de bismuth ($BiCl_3$) et le bromure de bismuth ($BiBr_3$), l'acétate de cadmium [$Cd(C_2H_3O_2)_2$], le benzoate de cadmium, l'oxalate de cadmium, le salycilate de cadmium, l'acétate de bismuth [$Bi(C_2H_3O_2)_3$], le benzoate de bismuth, l'acétate mercurique [$Hg(C_2H_3O_2)_2$], le benzoate mercurique, l'oxalate mercurique, l'acétate de magnésium, le benzoate de magnésium, le laurate de magnésium, l'oxalate de magnésium, le palmitate de magnésium, le stéarate de magnésium.

Les exemples suivants illustrent l'invention et montrent comment elle peut être mise en pratique.

*Exemples 1 à 3:*

Dans un ballon en verre de 100 ml équipé d'un thermomètre, d'un réfrigérant ascendant et d'un système de chauffage, on charge 53,3 g d'éthoxyméthylènemalonate d'éthyle, des quantités variables de stabilisant, et 800 ppm (partie en poids par million) de $ZnCl_2$. On porte ensuite le contenu du ballon à 155° C et le maintient 8 heures à cette température. A titre comparatif on procède à la même expérience en absence d'inhibiteur, en présence et en absence de $ZnCl_2$. Après refroidissement du contenu du ballon on dose l'éthoxyméthylènemalonate d'éthyle par chromatographie en phase gazeuse.

On a obtenu les résultats consignés dans le tableau suivant:

| Ex. | Stabilisant | | EMME (1) Récupéré | |
|---|---|---|---|---|
| | Nature | Quantité en g | En poids | En % (2) |
| 1 | Phosphate acide d'éthyle (3) | 0,36 | 52,74 | 99 |
| 2 | Hydroxy-8 quinoléine | 0,44 | 53,3 | 100 |
| 3 | Hydroxy-8 quinoléine | 0,177 | 52,05 | 97,7 |
| Essais témoins | | | | |
| A (4) | néant | | 48,77 | 91,5 |
| B (5) | néant | | 53,3 | 100 |

(1) EMME éthoxyméthylènemalonate d'éthyle;
(2) par rapport à l'EMME chargé;
(3) mélange à 1,5% en moles de $H_3PO_4$; 45,2% de phosphate de monoéthyle; de 45,7% de phosphate de diéthyle; de 1,8% de phosphate triméthyle et 5,7% de pyrophosphates;
(4) essai réalisé en présence de $ZnCl_2$;
(5) essai réalisé en absence de $ZnCl_2$.

La comparaison de l'essai A avec l'essai B montre que $ZnCl_2$ est responsable de la dégradation de 8,5% de l'éthoxyméthylènemalonate d'éthyle et celle des exemples 1 à 4 fait ressortir l'influence des stabilisants sur l'action néfaste de $ZnCl_2$.

*Exemple 4:*

Dans une chaudière en acier inoxydable de 1,5 litre de capacité, équipée d'un thermomètre d'une colonne en acier inoxydable ayant de 5 à 7 plateaux théoriques et reliée à une pompe à vide on charge 500 g d'éthoxyméthylènemalonate de diéthyle, 1,344 g d'acétate de zinc dihydraté et 4,44 g d'hydroxy-8 quinoléine. On procède ensuite à la distillation de l'éthoxyméthylènemalonate sous une pression réduite à 38-40 mm de mercure. La distillation dure 8 heures au cours desquelles on recueille: une première fraction de 11,4 g contenant 94,5% d'EMME; une deuxième fraction de 451,1 g contenant 100% d'EMME; une troisième fraction de 18,9 g contenant 98,3% et un résidu de 10,2 g contenant 93% d'EMME. Au total on a retrouvé 98% de l'EMME chargé (490 g).

A titre comparatif on a répété la distillation en absence d'hydroxy-8 quinoléine et en présence de chlorure de zinc (essai C) et en absence d'acétate de zinc et de stabilisant (essai D). On a obtenu les résultats consignés dans le tableau suivant:

| | | Essai C | Essai D |
|---|---|---|---|
| Fraction | Quantité en g | 11,9 | 8,3 |
| | % en poids d'EMME | 95,2 | 90,3 |
| Fraction 2 | Quantité en g | 432,8 | 466,7 |
| | % en poids d'EMME | 99,3 | 100 |
| Fraction 3 | Quantité en g | 28 | |
| | % en poids d'EMME | 97 | |
| Résidu | Quantité en g | 14,7 | 17 |
| | % en poids d'EMME | 62,6 | 93 |
| EMME total récupéré | en poids | 477,4 | 490 |
| | en % | 95,4 | 98 |

*Exemple 5:*

On a procédé comme à l'exemple 1 en chauffant à 157°C pendant 7 h 30 min 54,12 g d'éthoxyméthylènemalonate d'éthyle contenant 0,167 g d'acétate de cadmium dihydraté et 0,36 g du phosphate de diéthyle défini à l'exemple 1.

Après arrêt du chauffage on dose 53,7 g d'EMME soit 99,2% de la quantité initiale.

A titre comparatif on a reproduit cet exemple en l'absence de phosphate. On a dosé 52,14 g d'EMME pour 54,54 g chargés, soit 95,6% de la quantité initiale.

*Exemple 6:*

On a procédé comme à l'exemple 1 en chauffant à 155°C pendant 7 h 30 min 53,29 g d'éthoxyméthylènemalonate d'éthyle contenant 0,412 g de $MgCl_2$, 6 $H_2O$ et 1,16 g du phosphate de diéthyle défini à l'exemple 1.

Après arrêt du chauffage on dose 48,92 g d'EMME soit 91,8% de la quantité initiale.

A titre comparatif on a reproduit cet exemple en l'absence de phosphate. On a dosé 37,1 g d'EMME pour 52,54 g chargés, soit 95,6% de la quantité initiale.

## Revendications

1. Procédé de stabilisation thermique des alkoxyalkylidènemalonates d'alkyles contenant des dérivés métalliques utilisés comme catalyseurs au cours de leur préparation par condensation entre un malonate et un orthoester, caractérisé en ce qu'on leur ajoute une quantité efficace d'un composé pris dans le groupe formé par les hydroxy-8 quinoléines et les phosphates acides organiques.

2. Procédé selon la revendication 1, caractérisé en ce qu'on l'applique à la distillation des alkoxyalkylidènemalonates d'alkyles contenant des dérivés métalliques.

3. Procédé selon la revendication 2, caractérisé en ce que les alkoxyalkylidènemalonates d'alkyles sont distillés à partir des mélanges réactionnels de condensation des malonates d'alkyle avec les orthoesters en présence de dérivés métalliques.

4. Procédé selon l'une quelconque des revendications 1 à 3, caractérisé en ce qu'on utilise comme phosphates acides organiques des composés de formule générale:

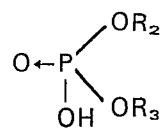

(I)

dans laquelle $R_2$ représente un radical hydrocarboné ayant de 1 à 20 atomes de carbone et $R_3$ un atome d'hydrogène ou un radical tel que $R_2$ et qui peut dans ce cas lui être identique ou différent.

5. Procédé selon l'une quelconque des revendications 1 à 3, caractérisé en ce qu'on utilise comme

stabilisant une hydroxy-8 quinoléine de formule générale:

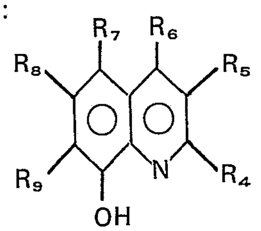

(II)

dans laquelle $R_4$ à $R_9$ identiques ou différents représentent un atome d'hydrogène ou des radicaux hydrocarbonés comportant de 1 à 20 et de préférence de 1 à 6 atomes de carbone, un atome d'halogène ou les groupes $-SO_3H$, $-NO_2$, $-COOH$.

6. Procédé selon l'une quelconque des revendications 1 à 5, caractérisé en ce que l'on utilise comme stabilisant l'hydroxy-8 quinoléine et le phosphate acide d'éthyle.

## Patentansprüche

1. Verfahren zur thermischen Stabilisierung von Alkyl-Alkoxyalkylidenmalonaten, die metallische Derivate enthalten, die im Verlauf ihrer Herstellung durch Kondensation aus einem Malonat und einem Orthoester als Katalysator verwendet wurden, dadurch gekennzeichnet, dass man diesen eine wirksame Menge einer Verbindung ausgewählt aus der Gruppe der 8-Hydroxychinoline und der sauren organischen Phosphate zusetzt.

2. Verfahren nach Anspruch 1, dadurch gekennzeichnet, dass man es bei der Destillation der Alkyl-Alkoxyalkylidenmalonate, die metallische Derivate enthalten, anwendet.

3. Verfahren nach Anspruch 2, dadurch gekennzeichnet, dass die Alkyl-Alkoxyalkylidenmalonate aus den Reaktionsmischungen der Kondensation der Alkylmalonate mit den Orthoestern in Gegenwart von metallischen Derivaten abdestilliert werden.

4. Verfahren nach einem der Ansprüche 1 bis 3, dadurch gekennzeichnet, dass man als organische Phosphorsäuren Verbindungen der allgemeinen Formel:

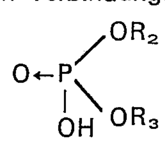

(I)

verwendet, in der $R_2$ einen Kohlenwasserstoffrest mit 1 bis 20 Kohlenstoffatomen darstellt und $R_3$ ein Wasserstoffatom oder einen Rest der gleichen Art wie $R_2$ darstellt, der in diesem Fall identisch oder verschieden sein kann.

5. Verfahren nach einem der Ansprüche 1 bis 3, dadurch gekennzeichnet, dass man als Stabilisator ein 8-Hydroxychinolin der allgemeinen Formel:

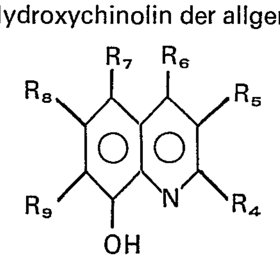

(II)

verwendet, in der $R_4$ bis $R_9$ identisch oder verschieden sind und ein Wasserstoffatom oder Kohlenwasserstoffreste mit 1 bis 20 und vorzugsweise 1 bis 6 Kohlenstoffatomen, ein Halogenatom oder die Gruppen $-SO_3H$, $-NO_2$, $-COOH$ darstellen.

6. Verfahren nach einem der Ansprüche 1 bis 5, dadurch gekennzeichnet, dass man als Stabilisator 8-Hydroxychinolin und das saure Ethylphosphat verwendet.

## Claims

1. Process for heat stabilization of alkyl alkoxyalkylidenemalonates containing metal derivatives employed as catalysts during their preparation by condensation of a malonate with an ortho ester, characterized in that an effective quantity of a compound taken from the group consisting of 8-hydroxyquinolines and organic hydrogen phosphates is added thereto.

2. Process according to Claim 1, characterized in that it is applied to the distillation of alkyl alkoxyalkylidenemalonates containing metal derivatives.

3. Process according to Claim 2, characterized in that the alkyl alkoxyalkylidenemalonates are distilled from reaction mixtures from the condensation of alkyl malonates with ortho esters in the presence of metal derivatives.

4. Process according to any one of Claims 1 to 3, characterized in that the organic hydrogen phosphates used are compounds of general formula:

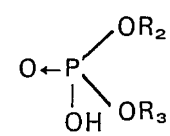

(I)

in which $R_2$ denotes a hydrocarbon radical containing from 1 to 20 carbon atoms and $R_3$ a hydrogen atom or a radical such as $R_2$ and which can in this case be identical to it or different.

5. Process according to any one of Claims 1 to 3, characterized in that the stabilizer used is an hydroxyquinoline of general formula:

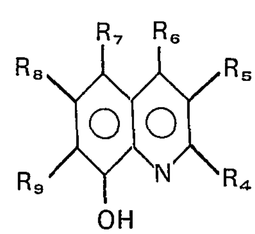

(II)

in which $R_4$ to $R_9$, which are identical or different, denote a hydrogen atom or hydrocarbon radicals containing from 1 to 20 and preferably from 1 to 6 carbon atoms, a halogen atom or the groups $-SO_3H$, $-NO_2$ and $-COOH$.

6. Process according to any one of Claims 1 to 5, characterized in that the stabilizer used is 8-hydroxyquinoline and ethyl hydrogen phosphate.